(19) Europäisches Patentamt

European Patent Office

Office européen des brevets

(11) **EP 1 347 049 A1**

(12) **EUROPEAN PATENT APPLICATION**
published in accordance with Art. 158(3) EPC

(43) Date of publication:
24.09.2003 Bulletin 2003/39

(51) Int Cl.7: **C12N 15/09**, C12N 15/12,
C07K 14/705, C12N 1/15,
C12N 1/19, C12N 1/21,
C12N 5/10, C12Q 1/02,
C07K 16/18

(21) Application number: 01271874.8

(22) Date of filing: 25.12.2001

(86) International application number:
PCT/JP01/11330

(87) International publication number:
WO 02/052000 (04.07.2002 Gazette 2002/27)

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **26.12.2000 JP 2000396020**

(71) Applicant: **YAMANOUCHI PHARMACEUTICAL
CO. LTD.
Tokyo 103-8411 (JP)**

(72) Inventors:
• **YOKOI, Hiromichi,
c/o Yamanouchi Pharm. Co. Ltd.
Tsukuba-shi, Ibaraki 305-8585 (JP)**

• **INAMURA, Kohei,
c/o Yamanouchi Pharma. Co. Ltd.
Tsukuba-shi, Ibaraki 305-8585 (JP)**
• **SANO, Yorikata,
c/o Yamanouchi Pharma. Co. Ltd.
Tsukuba-shi, Ibaraki 305-8585 (JP)**
• **MIYAKE, Akira,
c/o Yamanouchi Pharma. Co. Ltd.
Tsukuba-shi, Ibaraki 305-8585 (JP)**
• **MOCHIZUKI, Shinobu,
c/o Yamanouchi Pharma. Co Ltd.
Tsukuba-shi, Ibaraki 305-8585 (JP)**

(74) Representative: **HOFFMANN - EITLE
Patent- und Rechtsanwälte
Arabellastrasse 4
81925 München (DE)**

(54) **NOVEL POTASSIUM CHANNEL**

(57)    A novel polypeptide, a polynucleotide encoding the polypeptide, an expression vector comprising the polynucleotide, a cell transfected with the expression vector, an antibody binding to the polypeptide, a method for screening an agent for suppressing the polypeptide, a method for screening an antiobestic agent, and a process for producing the polypeptide are disclosed.

   The polypeptide is a novel potassium channel expressed in the hypothalamus.

SEQUENCE LISTING

<110> Yamanouchi Pharmaceutical Co., Ltd.

<120> Novel potassium channel

<130> YO133PCT-666

<150> JP 2000-396020
<151> 2000-12-26

<160> 6

<210> 1
<211> 1644
<212> DNA
<213> Homo sapiens

<220>
<221> CDS
<222> (13)..(1644)

<400> 1
```
ggcaacgaag ca atg aaa ttt cca atc gag acg cca aga aaa cag gtg aac   51
              Met Lys Phe Pro Ile Glu Thr Pro Arg Lys Gln Val Asn
                1               5                   10

tgg gat cct aaa gtg gcc gtt ccc gca gca gca ccg gtg tgc cag ccc    99
Trp Asp Pro Lys Val Ala Val Pro Ala Ala Ala Pro Val Cys Gln Pro
  15                  20                  25

aag agc gcc act aac ggg caa ccc ccg gct ccg gct ccg act cca act   147
Lys Ser Ala Thr Asn Gly Gln Pro Pro Ala Pro Ala Pro Thr Pro Thr
  30                  35                  40                  45

ccg cgc ctg tcc att tcc tcc cga gcc aca gtg gta gcc agg atg gaa   195
Pro Arg Leu Ser Ile Ser Ser Arg Ala Thr Val Val Ala Arg Met Glu
              50                  55                  60

ggc acc tcc caa ggg ggc ttg cag acc gtc atg aag tgg aag acg gtg   243
Gly Thr Ser Gln Gly Gly Leu Gln Thr Val Met Lys Trp Lys Thr Val
                  65                  70                  75

gtt gcc atc ttt gtg gtt gtg gtg gtc tac ctt gtc act ggc ggt ctt   291
Val Ala Ile Phe Val Val Val Val Val Tyr Leu Val Thr Gly Gly Leu
                  80                  85                  90
```

```
gtc ttc cgg gca ttg gag cag ccc ttt gag agc agc cag aag aat acc    339
Val Phe Arg Ala Leu Glu Gln Pro Phe Glu Ser Ser Gln Lys Asn Thr
         95                  100                 105


atc gcc ttg gag aag gcg gaa ttc ctg cgg gat cat gtc tgt gtg agc    387
Ile Ala Leu Glu Lys Ala Glu Phe Leu Arg Asp His Val Cys Val Ser
110                 115                 120                 125


ccc cag gag ctg gag acg ttg atc cag cat gct ctt gat gct gac aat    435
Pro Gln Glu Leu Glu Thr Leu Ile Gln His Ala Leu Asp Ala Asp Asn
                 130                 135                 140


gcg gga gtc agt cca ata gga aac tct tcc aac aac agc agc cac tgg    483
Ala Gly Val Ser Pro Ile Gly Asn Ser Ser Asn Asn Ser Ser His Trp
                 145                 150                 155


gac ctc ggc agt gcc ttt ttc ttt gct gga act gtc att acg acc ata    531
Asp Leu Gly Ser Ala Phe Phe Phe Ala Gly Thr Val Ile Thr Thr Ile
             160                 165                 170


ggg tat ggg aat att gct ccg agc act gaa gga ggc aaa atc ttt tgt    579
Gly Tyr Gly Asn Ile Ala Pro Ser Thr Glu Gly Gly Lys Ile Phe Cys
             175                 180                 185


att tta tat gcc atc ttt gga att cca ctc ttt ggt ttc tta ttg gct    627
Ile Leu Tyr Ala Ile Phe Gly Ile Pro Leu Phe Gly Phe Leu Leu Ala
190                 195                 200                 205


gga att gga gac caa ctt gga acc atc ttt ggg aaa agc att gca aga    675
Gly Ile Gly Asp Gln Leu Gly Thr Ile Phe Gly Lys Ser Ile Ala Arg
                 210                 215                 220


gtg gag aag gtg ttt cga aaa aag caa gtg agt cag acc aag atc cgg    723
Val Glu Lys Val Phe Arg Lys Lys Gln Val Ser Gln Thr Lys Ile Arg
                 225                 230                 235


gtc atc tca acc atc ctg ttc atc ttg gcc ggc tgc att gtg ttt gtg    771
Val Ile Ser Thr Ile Leu Phe Ile Leu Ala Gly Cys Ile Val Phe Val
                 240                 245                 250


acg atc cct gct gtc atc ttt aag tac atc gag ggc tgg acg gcc ttg    819
Thr Ile Pro Ala Val Ile Phe Lys Tyr Ile Glu Gly Trp Thr Ala Leu
         255                 260                 265


gag tcc att tac ttt gtg gtg gtc act ctg acc acg gtg ggc ttt ggt    867
```

Glu Ser Ile Tyr Phe Val Val Val Thr Leu Thr Thr Val Gly Phe Gly
270            275              280              285

gat ttt gtg gca ggg gga aac gct ggc atc aat tat cgg gag tgg tat      915
Asp Phe Val Ala Gly Gly Asn Ala Gly Ile Asn Tyr Arg Glu Trp Tyr
            290              295              300

aag ccc cta gtg tgg ttt tgg atc ctt gtt ggc ctt gcc tac ttt gca      963
Lys Pro Leu Val Trp Phe Trp Ile Leu Val Gly Leu Ala Tyr Phe Ala
            305              310              315

gct gtc ctc agt atg atc gga gat tgg cta cgg gtt ctg tcc aaa aag      1011
Ala Val Leu Ser Met Ile Gly Asp Trp Leu Arg Val Leu Ser Lys Lys
            320              325              330

aca aaa gaa gag gtg ggt gaa atc aag gcc cat gcg gca gag tgg aag      1059
Thr Lys Glu Glu Val Gly Glu Ile Lys Ala His Ala Ala Glu Trp Lys
            335              340              345

gcc aat gtc acg gct gag ttc cgg gag aca cgg cga agg ctc agc gtg      1107
Ala Asn Val Thr Ala Glu Phe Arg Glu Thr Arg Arg Arg Leu Ser Val
350              355              360              365

gag atc cac gat aag ctg cag cgg gca gcc acc atc cgc agc atg gag      1155
Glu Ile His Asp Lys Leu Gln Arg Ala Ala Thr Ile Arg Ser Met Glu
            370              375              380

cgc cgg cgg ctg ggc ctg gac cag cgg gcc cac tca ctg gac atg ctg      1203
Arg Arg Arg Leu Gly Leu Asp Gln Arg Ala His Ser Leu Asp Met Leu
            385              390              395

tcc ccc gag aag cgc tct gtc ttt gct gcc ctg gac acc ggc cgc ttc      1251
Ser Pro Glu Lys Arg Ser Val Phe Ala Ala Leu Asp Thr Gly Arg Phe
            400              405              410

aag gcc tca tcc cag gag agc atc aac aac cgg ccc aac aac ctg cgc      1299
Lys Ala Ser Ser Gln Glu Ser Ile Asn Asn Arg Pro Asn Asn Leu Arg
            415              420              425

ctg aag ggg ccg gag cag ctg aac aag cat ggg cag ggt gcg tcc gag      1347
Leu Lys Gly Pro Glu Gln Leu Asn Lys His Gly Gln Gly Ala Ser Glu
430              435              440              445

gac aac atc atc aac aag ttc ggg tcc acc tcc aga ctc acc aag agg      1395
Asp Asn Ile Ile Asn Lys Phe Gly Ser Thr Ser Arg Leu Thr Lys Arg
            450              455              460

```
aaa aac aag gac ctc aaa aag acc ttg ccc gag gac gtt cag aaa atc    1443
Lys Asn Lys Asp Leu Lys Lys Thr Leu Pro Glu Asp Val Gln Lys Ile
            465                 470                 475

tac aag acc ttc cgg aat tac tcc ctg gac gag gag aag aaa gag gag    1491
Tyr Lys Thr Phe Arg Asn Tyr Ser Leu Asp Glu Glu Lys Lys Glu Glu
            480                 485                 490

gag acg gaa aag atg tgt aac tca gac aac tcc agc aca gcc atg ctg    1539
Glu Thr Glu Lys Met Cys Asn Ser Asp Asn Ser Ser Thr Ala Met Leu
            495                 500                 505

acg gac tgt atc cag cag cac gct gag ttg gag aac gga atg ata ccc    1587
Thr Asp Cys Ile Gln Gln His Ala Glu Leu Glu Asn Gly Met Ile Pro
510                 515                 520                 525

acg gac acc aaa gac cgg gag ccg gag aac aac tca tta ctt gaa gac    1635
Thr Asp Thr Lys Asp Arg Glu Pro Glu Asn Asn Ser Leu Leu Glu Asp
                530                 535                 540

aga aac taa                                                        1644
Arg Asn
```

<210> 2
<211> 543
<212> PRT
<213> Homo sapiens

<400> 2

```
Met Lys Phe Pro Ile Glu Thr Pro Arg Lys Gln Val Asn Trp Asp Pro
  1           5                  10                  15
Lys Val Ala Val Pro Ala Ala Ala Pro Val Cys Gln Pro Lys Ser Ala
            20                  25                  30
Thr Asn Gly Gln Pro Pro Ala Pro Ala Pro Thr Pro Thr Pro Arg Leu
            35                  40                  45
Ser Ile Ser Ser Arg Ala Thr Val Val Ala Arg Met Glu Gly Thr Ser
        50                  55                  60
Gln Gly Gly Leu Gln Thr Val Met Lys Trp Lys Thr Val Val Ala Ile
65                  70                  75                  80
Phe Val Val Val Val Val Tyr Leu Val Thr Gly Gly Leu Val Phe Arg
                85                  90                  95
Ala Leu Glu Gln Pro Phe Glu Ser Ser Gln Lys Asn Thr Ile Ala Leu
            100                 105                 110
Glu Lys Ala Glu Phe Leu Arg Asp His Val Cys Val Ser Pro Gln Glu
```

```
            115                  120                  125
Leu Glu Thr Leu Ile Gln His Ala Leu Asp Ala Asp Asn Ala Gly Val
        130                  135                  140
Ser Pro Ile Gly Asn Ser Ser Asn Asn Ser Ser His Trp Asp Leu Gly
145                  150                  155                  160
Ser Ala Phe Phe Phe Ala Gly Thr Val Ile Thr Thr Ile Gly Tyr Gly
                165                  170                  175
Asn Ile Ala Pro Ser Thr Glu Gly Gly Lys Ile Phe Cys Ile Leu Tyr
            180                  185                  190
Ala Ile Phe Gly Ile Pro Leu Phe Gly Phe Leu Leu Ala Gly Ile Gly
            195                  200                  205
Asp Gln Leu Gly Thr Ile Phe Gly Lys Ser Ile Ala Arg Val Glu Lys
            210                  215                  220
Val Phe Arg Lys Lys Gln Val Ser Gln Thr Lys Ile Arg Val Ile Ser
225                  230                  235                  240
Thr Ile Leu Phe Ile Leu Ala Gly Cys Ile Val Phe Val Thr Ile Pro
                245                  250                  255
Ala Val Ile Phe Lys Tyr Ile Glu Gly Trp Thr Ala Leu Glu Ser Ile
            260                  265                  270
Tyr Phe Val Val Val Thr Leu Thr Thr Val Gly Phe Gly Asp Phe Val
            275                  280                  285
Ala Gly Gly Asn Ala Gly Ile Asn Tyr Arg Glu Trp Tyr Lys Pro Leu
            290                  295                  300
Val Trp Phe Trp Ile Leu Val Gly Leu Ala Tyr Phe Ala Ala Val Leu
305                  310                  315                  320
Ser Met Ile Gly Asp Trp Leu Arg Val Leu Ser Lys Lys Thr Lys Glu
                325                  330                  335
Glu Val Gly Glu Ile Lys Ala His Ala Ala Glu Trp Lys Ala Asn Val
            340                  345                  350
Thr Ala Glu Phe Arg Glu Thr Arg Arg Arg Leu Ser Val Glu Ile His
            355                  360                  365
Asp Lys Leu Gln Arg Ala Ala Thr Ile Arg Ser Met Glu Arg Arg Arg
            370                  375                  380
Leu Gly Leu Asp Gln Arg Ala His Ser Leu Asp Met Leu Ser Pro Glu
385                  390                  395                  400
Lys Arg Ser Val Phe Ala Ala Leu Asp Thr Gly Arg Phe Lys Ala Ser
                405                  410                  415
Ser Gln Glu Ser Ile Asn Asn Arg Pro Asn Asn Leu Arg Leu Lys Gly
            420                  425                  430
Pro Glu Gln Leu Asn Lys His Gly Gln Gly Ala Ser Glu Asp Asn Ile
            435                  440                  445
Ile Asn Lys Phe Gly Ser Thr Ser Arg Leu Thr Lys Arg Lys Asn Lys
            450                  455                  460
Asp Leu Lys Lys Thr Leu Pro Glu Asp Val Gln Lys Ile Tyr Lys Thr
465                  470                  475                  480
Phe Arg Asn Tyr Ser Leu Asp Glu Glu Lys Lys Glu Glu Glu Thr Glu
```

```
                    485                  490                  495
Lys Met Cys Asn Ser Asp Asn Ser Ser Thr Ala Met Leu Thr Asp Cys
                500                  505                  510
Ile Gln Gln His Ala Glu Leu Glu Asn Gly Met Ile Pro Thr Asp Thr
            515                  520                  525
Lys Asp Arg Glu Pro Glu Asn Asn Ser Leu Leu Glu Asp Arg Asn
        530                  535                  540
```

<210> 3
<211> 24
<212> DNA
<213> Artificial Sequence

<220>
<223> Description of Artificial Sequence: an
     artificially synthesized primer sequence

<400> 3
ggaattccgg caacgaagca atga                                    24


<210> 4
<211> 31
<212> DNA
<213> Artificial Sequence

<220>
<223> Description of Artificial Sequence: an
     artificially synthesized primer sequence

<400> 4
cggatccgtt agtttctgtc ttcaagtaat g                            31


<210> 5
<211> 28
<212> DNA
<213> Homo sapiens

<400> 5
gaaggcacct cccaaggggg cttgcaga                                28


<210> 6

```
<211> 28
<212> DNA
<213> Homo sapiens

<400> 6
gtgggtatca ttccgttctc caactcag                          28
```

## Description

### TECHNICAL FIELD

This invention relates to a novel potassium channel.

### BACKGROUND ART

An ob gene is specifically expressed in an adipose tissue and encodes leptin. Leptin is sent to the central nerve system, particularly the hypothalamus, as information derived from a peripheral tissue which controls a fat accumulation, and suppresses an eating action by binding to a leptin receptor (Ob-R) present in each nucleus in the hypothalamus. Further, leptin induces an energy consumption in peripheral tissues via the autonomic nervous system. Expression of the leptin receptor in the hypothalamus is observed in nucleus paraventricularis, arcuate nucleus, ventromedial hypothalamic nucleas, and ventral anteromammillary nucleus. Further, elucidation of an action site or action mechanism of leptin is progressing, and it has been clarified that leptin inhibits neuropeptide Y (NPY) activities of inducing feeding and of suppressing the energy consumption in nerve cell bodies of arcuate nucleus, and enhances activities of suppressing feeding and of inducing the energy consumption via a melanocortin-4 receptor (MC-4R) which is a receptor of the melanocyte-stimulating hormone ($\alpha$-MSH) (FEBS Letter, 387, p.113, 1996; Diabetes, 45, p.531, 1996; and Nature, 385, p.165, 1997).

Further, the hypothalamus controls feeding through chemical information in blood, or neural information sent from chemoreceptors or mechanoreceptors in peripheral digestive organs. It has been long known that medial hypothalamic area consisting of ventromedial hypothalamic nucleas, nucleus paraventricularis, supla chiasmatic nucleus), and the like act as a center in a feed-suppressing mechanism (for example, its destruction causes overeating and/or fatness, or a feeding suppression is observed by stimulation) (Shimizu N. et al., Brain Res., 416, 153-156, 1987; and Takaki A. et al., Am. J. Physiol., 262, R586-R594, 1987). Therefore, it is considered that an increase of a neural excitation in the medial hypothalamic area has an effect on the feeding suppression.

The excitation in neurons changes according to a membrane potential. When the membrane potential is depolarized, the neuron is excited. Conversely, when the membrane potential is hyperpolarized, the excitation decreases. As such, it is possible to control the excitation of the neuron by controlling the membrane potential thereof. The membrane potential is determined by the distribution of charges from inorganic ions such as $Na^+$, $K^+$, $Cl^-$, $Ca^{2+}$, or the like inside and outside of a cell. The charge distribution is changed by an opening and closing of various ion channels composed of membrane proteins. Therefore, the opening and closing of ion channels play an important role in the change of the membrane potential. In particular, it is considered that a potassium channel (a membrane protein which allows a potassium ion to selectively pass through) is involved with the maintenance of a resting membrane potential, the repolarization of a membrane potential after depolarization, or the like, and the potassium channel is a desirable target for controlling the excitation of neurons, and an agent for suppressing it increases the membrane potential of cells and the excitation of neurons (Hille, B., Ionic Channels of Excitable Membranes, 2nd Ed., Sinauer Associates Inc., MA, 1992; Edward et al., THE ION CHANNEL Fact Book, Academic Press, 1999).

### DISCLOSURE OF INVENTION

The object of the present invention is to provide a novel potassium channel protein, and a novel polynucleotide encoding the protein, and to provide a convenient screening system to obtain a substance useful as an antiobestic agent in which the mechanism is an increase in the excitation of hypothalamus neurons.

The present inventors have conducted intensive studies and, as a result, obtained a polynucleotide encoding a potassium channel consisting of the amino acid sequence of SEQ ID NO: 2 and exclusively expressed in the hypothalamus controlling the feeding, prepared the channel, and provided a screening tool for an antiobestic agent. The present inventors confirmed that, among the hypothalamus, the channel is expressed in nucleus paraventricularis, arcuate nucleus, and ventromedial hypothalamic nucleas, in which a leptin receptor having activities suppressing feeding and inducing an energy consumption is similarly expressed, and thus clarified that the channel is useful as a screening tool for an antiobestic agent. Further, the potassium channel was expressed, a system for detecting the channel activity was constructed, and a method for screening an agent for suppressing the polypeptide of the present invention and a method for screening an antiobestic agent were provided, and the present invention was completed.

Accordingly, the present invention relates to:

[1] (1) a polypeptide consisting of an amino acid sequence of SEQ ID NO: 2, or (2) a polypeptide exhibiting a potassium channel activity and consisting of an amino acid sequence in which one or several amino acids are substituted, deleted, inserted, and/or added in an amino acid sequence of SEQ ID NO: 2;

[2] a polypeptide consisting of an amino acid sequence of SEQ ID NO: 2;
[3] a polynucleotide encoding the polypeptide of the item [1] or [2];
[4] an expression vector comprising the polynucleotide of the item [3];
[5] a cell transfected with the expression vector of the item [4];
[6] a method for screening an agent for suppressing the polypeptide of the item [1] or [2], comprising the steps of:

bringing a cell expressing the polypeptide into contact with a compound to be tested, and
analyzing whether or not the polypeptide is suppressed;

[7) a method for screening an antiobestic agent, comprising the steps of:

bringing a cell expressing the polypeptide of the item [1] or [2] into contact with a compound to be tested, and
analyzing whether or not the polypeptide is suppressed;

[8] a process for producing the polypeptide of the item [1] or [2], comprising the steps of:

culturing the cell of the item [5], and
recovering the polypeptide; and

[9] an antibody or a fragment thereof, which binds to the polypeptide of the item [1] or [2].

BRIEF DESCRIPTION OF DRAWINGS

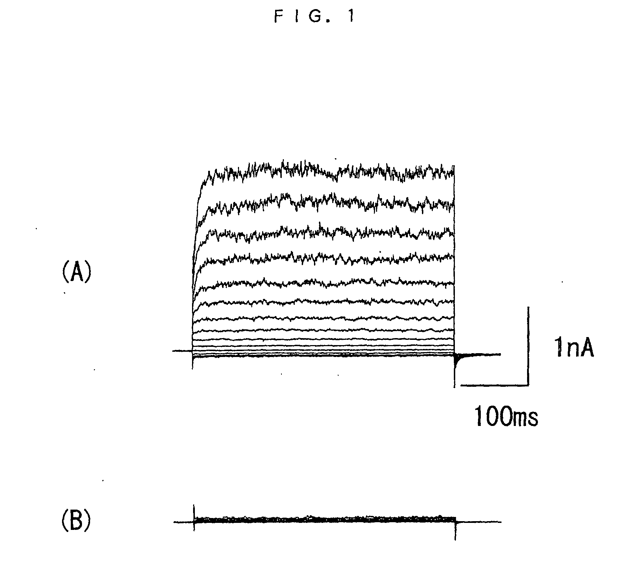

Fig. 1 is a graph showing an observational result of an outward current induced when a depolarization pulse was given to an L929 cell transfected with a plasmid pIRESneo2-543 (A) or a control vector (B) while voltage-clamping by a whole-cell voltage-clamp method.

BEST MODE FOR CARRYING OUT THE INVENTION

The present invention will be explained in detail hereinafter.

[1] The polypeptide of the present invention

The polypeptide of the present invention includes

(1) a polypeptide consisting of the amino acid sequence of SEQ ID NO: 2; and
(2) a polypeptide consisting of an amino acid sequence in which one or several amino acids in total are substituted, deleted, inserted, and/or added at one or plural positions in the amino acid sequence of SEQ ID NO: 2, and exhibiting a potassium channel activity (hereinafter referred to as a variation functionally equivalent).

As the polypeptide of the present invention, the polypeptide consisting of the amino acid sequence of SEQ ID NO: 2 is preferable.
The term "exhibiting a potassium channel activity" as used herein means that both two features, i.e.,

(a) that a current is generated by a voltage stimulus; and
(b) that the selectivity of a potassium ion is high, are met (Hille, B., Ionic Channels of Excitable Membranes, 2nd Ed., Sinauer Associates Inc., MA, 1992; or Edward et al., THE ION CHANNEL Fact Book, Academic Press, 1999).

Whether or not "a current is generated by a voltage stimulus" in a polypeptide (hereinafter referred to as a test polypeptide) as used herein may be confirmed by a method known to those skilled in the art (Hille, B., Ionic Channels of Excitable Membranes, 2nd Ed., Sinauer Associates Inc., MA, 1992). A method for confirming it is not particularly limited but, for example, the following method (preferably a method described in Example 4) may be used. More particularly, a cell is transfected with an expression vector comprising a polynucleotide encoding the test polypeptide, and a depolarization pulse of 0 mV from a holding potential of -80 mV for 400 msec is given to the resulting cell while voltage-clamping by a whole-cell voltage-clamp method. When an outward current is generated by the depolarization pulse, it may be confirmed that "a current is generated by a voltage stimulus" in the test polypeptide.
Whether or not "the selectivity of a potassium ion is high" in the test polypeptide as used herein may be confirmed by a method known to those skilled in the art (Hille, B., Ionic Channels of Excitable Membranes, 2nd Ed.,

Sinauer Associates Inc., MA, 1992). A method for confirming it is not particularly limited, but for example the following method (preferably a method described in Example 5) may be used. More particularly, a cell is transfected with an expression vector comprising a polynucleotide encoding the test polypeptide, and then the resulting cell is voltage-clamped by a whole-cell voltage-clamp method. While changing a potassium ion concentration in the extracellular solution, a reversal potential in each potassium ion concentration is measured using a voltage ramp. The reversal potential in each potassium ion concentration is plotted. When the slope of the resulting line is close to the theoretical value (preferably 47 to 58 mV/decade) of slope calculated from Nernst's equation:

$$E = (2.303RT/F)\log([K]out/[K]in)$$

(wherein E is a membrane potential, [K]out is a concentration of an extracellular potassium ion, [K]in is a concentration of an intracellular potassium ion, R is the gas constant, F is the Faraday constant, and T is an absolute temperature), it may be confirmed that "the selectivity of a potassium ion is high" in the test polypeptide.

The polypeptide consisting of the amino acid sequence of SEQ ID NO: 2, one of the polypeptides of the present invention, is a novel human potassium channel protein consisting of 543 amino acid residues. The polypeptide consisting of the amino acid sequence of SEQ ID NO: 2 is exclusively expressed in the hypothalamus which controls feeding as shown in Example 2, and is expressed, among the hypothalamus, in nucleus paraventricularis, arcuate nucleus, and ventromedial hypothalamic nucleas in which a leptin receptor suppressing feeding is expressed.

In this connection, J.B.C., 275, September 15, 28398-28405, 2000 discloses human TREK-2 (full-length: 538aa) which is a potassium channel and comprises a sequence consisting of the 18th to 543rd amino acids in the amino acid sequence of SEQ ID NO: 2 as the 13th to 538th amino acid sequence. However, it is confirmed that human TREK-2 is strongly expressed in the kidney and pancreas, and expressed in the testis, brain, large intestine, and small intestine. Further, J.B.C., 275, June 9, 17412-17419, 2000 discloses rat TREK-2 (full-length: 538aa) which is a potassium channel and comprises amino acid sequences having a 100% and 97% homology with those consisting of the 1st to 25th and the 44th to 543rd amino acids in the amino acid sequence of SEQ ID NO: 2 respectively, as the 1st to 25th and the 39th to 538th amino acid sequences, as shown by a result obtained by a BLAST (Basic local alignment search tool; Altschul, S. F. et al., J. Mol. Biol., 215, 403-410, 1990) search. However, it is confirmed that rat TREK-2 is strongly expressed in the cerebellum, and weekly expressed in the spleen and testis, as shown by a result obtained by a northern blotting method. Human TREK-2 and rat TREK-2 are not expressed in hypothalamus involved with the feeding, and particularly there is no disclosure that they are involved in the feeding. Therefore, it is considered that human TREK-2 and rat TREK-2 are not involved with a feeding-controlling function, and that their role in the body is different from that of the polypeptide of the present invention having the feeding-controlling function. As described above, the polypeptide of the present invention could not be easily obtained using TREK-2, and has an unexpected advantageous effect.

The variation functionally equivalent of the present invention is not particularly limited, so long as it is a polypeptide consisting of an amino acid sequence in which one or several amino acids (for example, 1 to 3 amino acids) in total are substituted, deleted, inserted, and/or added at one or plural positions (for example, 1 to 3 positions) in the amino acid sequence of SEQ ID NO: 2, and exhibiting the potassium channel activity. Further, an origin of the variation functionally equivalent is not limited to a human.

The variation functionally equivalent of the present invention includes, for example, not only human variations of the polypeptide consisting of the amino acid sequence of SEQ ID NO: 2, but also variations functionally equivalent derived from organisms other than a human (such as a mouse, a hamster, or a dog). Further, it includes polypeptides prepared using polynucleotides obtained by artificially modifying their amino acid sequences encoded thereby by genetic engineering techniques, on the basis of polynucleotides encoding these native polypeptides (i.e., human variations or variations functionally equivalent derived from organisms other than a human), or on the basis of polynucleotides encoding an amino acid sequence represented by the amino acid sequence of SEQ ID NO: 2. The term "variation" as used herein means an individual difference between the same polypeptides in the same species or a difference between homologous polypeptides in several species.

Human variations of the polypeptide consisting of the amino acid sequence of SEQ ID NO: 2 or variations functionally equivalent derived from organisms other than a human may be obtained by those skilled in the art in accordance with the information of a base sequence (for example, a sequence consisting of the 13th to 1644th bases in the base sequence of SEQ ID NO: 1) of a polynucleotide encoding the polypeptide consisting of the amino acid sequence of SEQ ID NO: 2. In this connection, genetic engineering techniques may be performed in accordance with known methods (for example, Maniatis, T. et al., "Molecular Cloning-A Laboratory Manual", Cold Spring Harbor Laboratory, NY, 1982), unless otherwise specified.

For example, an appropriate probe or appropriate primers are designed in accordance with the information of a base sequence of a polynucleotide encoding the polypeptide consisting of the amino acid sequence of SEQ ID NO: 2. A polymerase chain reaction (PCR) method (Saiki, R. K. et al., Science, 239, 487-491, 1988) or a hybridization method is carried out using a sample (for example, total RNA or an mRNA fraction, a cDNA library, or a phage library) prepared from an organism (for example, a mammal such as a human, a mouse, a hamster, or a dog) of interest and the primers or the probe to obtain a polynucleotide encoding the polypeptide. A desired polypeptide may be obtained by expressing the resulting polynucleotide in an appropriate expression system, and then, for example, by confirming that an outward current is generated by a depolarization pulse in the expressed polypeptide by a method described in Example 4, and further confirming that the selectivity of a potassium ion is high by a method described in Example 5.

Further, the polypeptide artificially modified by genetic engineering techniques may be obtained by, for example, the following procedure. A polynucleotide encoding the polypeptide is obtained by a conventional method such as site-directed mutagenesis (Mark, D. F. et al., Proc. Natl. Acad. Sci. USA, 81, 5662-5666, 1984). A desired polypeptide may be obtained by expressing the resulting polynucleotide in an appropriate expression system, and then, for example, by confirming that an outward current is generated by a depolarization pulse in the expressed polypeptide by a method described in Example 4, and further confirming that the selectivity of a potassium ion is high by a method described in Example 5.

[2] The polynucleotide of the present invention

The polynucleotide of the present invention is not particularly limited, so long as it encodes the polypeptide of the present invention. As the polynucleotide of the present invention, there may be mentioned, for example, a polynucleotide consisting of a sequence consisting of the 13th to 1644th bases in the base sequence of SEQ ID NO: 1. In this connection, the term "polynucleotide" as used herein includes both DNA and RNA.

A method for producing the polynucleotide of the present invention is not particularly limited, but there may be mentioned, for example, (1) a method using PCR, (2) a method using conventional genetic engineering techniques (i.e., a method for selecting a transformant comprising a desired cDNA from strains transformed with a cDNA library), or (3) a chemical synthesis method. These methods will be explained in this order hereinafter.

In the method using PCR of the item (1), the polynucleotide of the present invention may be produced, for example, by the following procedure.

mRNA is extracted from human cells or tissue capable of producing the polypeptide of the present invention. A pair of primers, between which full-length mRNA corresponding to the polypeptide of the present invention or a partial region of the mRNA is located, is synthesized on the basis of the base sequence of a polynucleotide encoding the polynucleotide of the present invention. Full-length cDNA encoding the polypeptide of the present invention or a part of the cDNA may be obtained by performing a reverse transcriptase-polymerase chain reaction (RT-PCR) using the extracted mRNA as a template.

More particularly, total RNA containing mRNA encoding the polypeptide of the present invention is extracted by a known method from cells or tissue capable of producing the polypeptide of the present invention. As an extraction method, there may be mentioned, for example, a guanidine thiocyanate-hot phenol method, a guanidine thiocyanateguanidine hydrochloride method, or a guanidine thiocyanate-cesium chloride method. The guanidine thiocyanate-cesium chloride method is preferably used. The cells or tissue capable of producing the polypeptide of the present invention may be identified, for example, by a northern blotting method using a polynucleotide or a part thereof encoding the polypeptide of the present invention or a western blotting method using an antibody specific for the polypeptide of the present invention.

Next, the extracted mRNA is purified. Purification of the mRNA may be made in accordance with a conventional method. For example, the mRNA may be purified by adsorption and elution using an oligo(dT)-cellulose column. The mRNA may be further fractionated by, for example, a sucrose density gradient centrifugation, if necessary. Alternatively, commercially available extracted and purified mRNA may be used without carrying out the extraction of the mRNA.

Next, the first-strand cDNA is synthesized by carrying out a reverse transcriptase reaction of the purified mRNA in the presence of a random primer, an oligo dT primer, and/or a custom primer. This synthesis may be carried out in accordance with a conventional method. The resulting first-strand cDNA is subjected to PCR using two primers between which a full-length or a partial region of the polynucleotide of interest is located, thereby amplifying the cDNA of interest. The resulting DNA is fractionated by, for example, an agarose gel electrophoresis. The DNA fragment of interest may be obtained by carrying out a digestion of the DNA with restriction enzymes and subsequent ligation, if necessary.

In the method using conventional genetic engineering techniques of the item (2), the polynucleotide of the present invention may be produced, for example, by the following procedure.

First, single-stranded cDNA is synthesized by using reverse transcriptase from mRNA prepared by the above-mentioned PCR method as a template, and then double-stranded cDNA is synthesized from the single-stranded cDNA. As this method, there may be mentioned, for example, an S1 nuclease method (Efstratiadis, A. et al., Cell, 7, 279-288, 1976), a Land method (Land, H. et al., Nucleic Acids Res., 9, 2251-2266, 1981), an O. Joon Yoo method (Yoo, O. J.

et al., Proc. Natl. Acad. Sci. USA, 79, 1049-1053, 1983), and an Okayama-Berg method (Okayama, H. and Berg, P., Mol. Cell. Biol., 2, 161-170, 1982).

Next, a recombinant plasmid comprising the double-stranded cDNA is prepared and introduced into an Escherichia coli strain, such as DH 5α, thereby transforming the strain. A transformant is selected using a drug resistance against, for example, tetracycline or ampicillin as a marker. When the host cell is E. coli, transformation of the host cell may be carried out, for example, by the method of Hanahan (Hanahan, D. J., Mol. Biol., 166, 557-580, 1983); namely, a method in which the recombinant DNA is added to competent cells prepared in the presence of $CaCl_2$, $MgCl_2$, or RbCl. Further, as a vector other than a plasmid, a phage vector such as a lambda system may be used.

As a method for selecting a transformant containing the cDNA of interest from the resulting transformants, various methods such as (i) a method for screening a transformant using a synthetic oligonucleotide probe, (ii) a method for screening a transformant using a probe produced by PCR, (iii) a method for screening a transformant using an antibody against the polypeptide of the present invention, or (iv) a method for screening a transformant on the basis of the potassium channel activity, may be used.

In the method of the item (i) for screening a transformant using a synthetic oligonucleotide probe, the transformant containing the cDNA of interest may be selected, for example, by the following procedure.

An oligonucleotide which corresponds to the whole or a part of the polypeptide of the present invention is synthesized (in this case, it may be either a nucleotide sequence taking the codon usage into consideration or a plurality of nucleotide sequences as a combination of possible nucleotide sequences) and, using this oligonucleotide as a probe (labeled with $^{32}$P or $^{33}$P), hybridized with a nitrocellulose filter on which DNAs of the transformants are denatured and fixed, to screen and select resulting positive strains.

In the method of the item (ii) for screening a transformant using a probe produced by PCR, the transformant containing the cDNA of interest may be selected, for example, by the following procedure.

Oligonucleotides of a sense primer and an antisense primer corresponding to a part of the polypeptide of the present invention are synthesized, and a DNA fragment encoding the whole or a part of the polypeptide of interest is amplified by carrying out PCR using these primers in combination. As a template DNA used in this method, cDNA synthesized by a reverse transcription reaction from mRNA of cells capable of producing the polypeptide of the present invention, or genomic DNA, may be used. The resulting DNA fragment is labeled with $^{32}$P or $^{33}$P, and a transformant containing the cDNA of interest is selected by carrying out a colony hybridization or a plaque hybridization using this fragment as a probe.

In the method of the item (iii) for screening a transformant using an antibody against the polypeptide of the present invention, the transformant containing the CDNA of interest may be selected, for example, by the following procedure.

First, cDNA is integrated into an expression vector, and polypeptides are produced on the cell surface of transformants. A transformant containing the cDNA of interest is selected by detecting a strain producing the desired polypeptide using an antibody against the polypeptide of the present invention and a second antibody against the first antibody.

In the method of the item (iv) for screening a transformant on the basis of the potassium channel activity, the transformant containing the cDNA of interest may be selected, for example, by the following procedure.

First, cDNA is integrated into an expression vector, and polypeptides are produced on the cell surface of transformants. A transformant containing the cDNA of interest is selected by detecting a strain producing the desired polypeptide on the basis of the potassium channel activity.

A method for collecting the polynucleotide of the present invention from the resulting transformant of interest can be carried out in accordance with a known method (for example, Maniatis, T. et al., "Molecular Cloning-A Laboratory Manual", Cold Spring Harbor Laboratory, NY, 1982). For example, it may be carried out by separating a fraction corresponding to the plasmid DNA from cells and cutting out the cDNA region from the plasmid DNA.

In the chemical synthesis method of the item (3), the polynucleotide of the present invention may be produced, for example, by binding DNA fragments produced by a chemical synthesis method. Each DNA can be synthesized using a DNA synthesizer [for example, Oligo 1000M DNA Synthesizer (Beckman) or 394 DNA/RNA Synthesizer (Applied Biosystems)].

Further, the polynucleotide of the present invention may be produced by nucleic acid chemical synthesis in accordance with a conventional method such as a phosphate triester method (Hunkapiller, M. et al., Nature, 10, 105-111, 1984), based on the information on the polypeptide of the present invention. In this connection, codons for each amino acid are known and can be optionally selected and determined by the conventional method, for example, by taking a codon usage of each host to be used into consideration (Crantham, R. et al., Nucleic Acids Res., 9, r43-r74, 1981). Further, a partial modification of codons of these base sequences can be carried out in accordance with a conventional method, such as site directed mutagenesis which uses a primer comprised of a synthetic oligonucleotide coding for a desired modification (Mark, D. F. et al., Proc. Natl. Acad. Sci. USA, 81, 5662-5666, 1984).

Determination of the DNA sequences obtained by the above-mentioned methods can be carried out by, for example, a Maxam-Gilbert chemical modification method (Maxam, A. M. and Gilbert, W., "Methods in Enzymology", 65, 499-559, 1980) or a dideoxynucleotide chain termination method (Messing, J. and Vieira, J., Gene, 19, 269-276, 1982).

[3] The expression vector and the cell of the present invention

An isolated polynucleotide of the present invention is re-integrated into an appropriate vector DNA and a eucaryotic or procaryotic host cell may be transfected by the resulting expression vector. Further, it is possible to express the polynucleotide in a desired host cell, by introducing an appropriate promoter and a sequence related to the gene expression into the vector.

The expression vector of the present invention is not particularly limited, so long as it comprises the polynucleotide of the present invention. As the expression vector, there may be mentioned, for example, an expression vector obtained by introducing the polynucleotide of the present invention into a known expression vector appropriately selected in accordance with a host cell to be used.

The cell of the present invention is not particularly limited, so long as it is transfected with the expression vector of the present invention and comprises the polynucleotide of the present invention. The cell of the present invention may be, for example, a cell in which the polynucleotide is integrated into a chromosome of a host cell, or a cell containing the polynucleotide as an expression vector comprising polynucleotide. Further, the cell of the present invention may be a cell expressing the polypeptide of the present invention, or a cell not expressing the polypeptide of the present invention. The cell of the present invention may be obtained by, for example, transfecting a desired host cell with the expression vector of the present invention.

In the eucaryotic host cells, for example, cells of vertebrates, insects, and yeast are included. As the vertebral cell, there may be mentioned, for example, a simian COS cell (Gluzman, Y., Cell, 23, 175-182, 1981), a dihydrofolate reductase defective strain of a Chinese hamster ovary cell (CHO) (Urlaub, G. and Chasin, L. A., Proc. Natl. Acad. Sci. USA, 77, 4216-4220, 1980), a CHO-K1 cell used in Example 6, a human embryonic kidney derived HEK293 cell, a 293-EBNA cell (Invitrogen) obtained by introducing an EBNA-1 gene of Epstein Barr Virus into HEK293 cell, a L929 cell (ATCC: CRL-2148) used in Example 3, or the like.

As an expression vector for a vertebral cell, a vector containing a promoter positioned upstream of the gene to be expressed, an RNA splicing site, a polyadenylation site, a transcription termination sequence, and the like may be generally used. The vector may further contain a replication origin, if necessary. As the expression vector, there may be mentioned, for example, pSV2dhfr containing an SV40 early promoter (Subramani, S. et al., Mol. Cell. Biol., 1, 854-864, 1981), pEF-BOS containing a human elongation factor promoter (Mizushima, S. and Nagata, S., Nucleic Acids Res., 18,5322, 1990), pCEP4 containing a cytomegalovirus promoter (Invitrogen), pIRESneo2 (CLONTECH), or the like.

When the COS cell is used as the host cell, a vector which has an SV40 replication origin, can perform an autonomous replication in the COS cell, and has a transcription promoter, a transcription termination signal, and an RNA splicing site, may be used as the expression vector. As the vector, there may be mentioned, for example, pME18S (Maruyama, K. and Takebe, Y., Med. Immunol., 20, 27-32, 1990), pEF-BOS (Mizushima, S. and Nagata, S., Nucleic Acids Res., 18, 5322, 1990), or pCDM8 (Seed, B., Nature, 329, 840-842, 1987).

The expression vector may be incorporated into COS cells by, for example, a DEAE-dextran method (Luthman, H. and Magnusson, G., Nucleic Acids Res., 11, 1295-1308, 1983), a calcium phosphate-DNA co-precipitation method (Graham, F. L. and van der Ed, A. J., Virology, 52, 456-457, 1973), a method using a commercially available transfection reagent (for example, FuGENE$^{TM}$6 Transfection Reagent; Roche Diagnostics), or an electroporation method (Neumann, E. et al., EMBO J., 1, 841-845, 1982).

When the CHO cell is used as the host cell, a transfected cell capable of stably producing the polypeptide of the present invention can be obtained by carrying out co-transfection of an expression vector comprising the polynucleotide encoding the polypeptide of the present invention, together with a vector capable of expressing a neo gene which functions as a G418 resistance marker, such as pRSVneo (Sambrook, J. et al., "Molecular Cloning-A Laboratory Manual", Cold Spring Harbor Laboratory, NY, 1989) or pSV2-neo (Southern, P. J. and Berg, P., J. Mol. Appl. Genet., 1, 327-341,1982), and selecting a G418 resistant colony.

When the 293-EBNA cell is used as the host cell, for example, pCEP4 (Invitrogen) containing a replication origin of Epstein Barr Virus and capable of performing an autonomous replication in the 293-EBNA cell may be used as the expression vector.

The cell of the present invention may be cultured in accordance with the conventional method [for example, "Shin Seikagaku Jikken Koza 18, Saibou Baiyou Gijyutsu (Japanese Biochemical Society)", Tokyo Kagaku Dojin, 1990], and the polypeptide of the present invention is produced in the cells or on the cell surface. As a medium to be used in the culturing, a medium commonly used in a desired host cell may be appropriately selected. In the case of the COS cell, for example, a medium such as an RPMI-1640 medium or a Dulbecco's modified Eagle's minimum essential medium (DMEM) may be used, by supplementing it with a serum component such as fetal bovine serum (FBS) if necessary. In the case of the 293-EBNA cell, a medium such as a Dulbecco's modified Eagle's minimum essential medium (DMEM) with a serum component such as fetal bovine serum (FBS) and G418 may be used.

The polypeptide of the present invention produced in the cells or on the cell surface of the present invention by culturing the cells may be separated and purified therefrom by various known separation techniques [for example, Okada, M. and Miyazaki K., "Kaitei, Tanpakushitsu Jikken Noto, Jyo·Ge (Revision, Notebook for Protein Experiments)", Yodosha 1999] making use of the physical properties, chemical properties and the like of the polypeptide. More particularly, for example, a cell membrane fraction containing the polypeptide of the present invention may be obtained by culturing the cell in which the polypeptide of the present invention is expressed on the surface thereof, suspending the cell in a buffer, homogenizing the suspension, and centrifuging the homogenate. The polypeptide of the present invention may be purified by solubilizing the resulting cell membrane fraction, and then by a treatment with a commonly used protein precipitant, ultrafiltration, various liquid chromatography techniques such as molecular sieve chromatography (gel filtration), adsorption chromatography, ion exchange chromatography, affinity chromatography, or high performance liquid chromatography (HPLC), or dialysis, or a combination thereof. In this connection, when the cell membrane fraction is solubilized using as mild as possible a solubilizing agent (such as CHAPS, Triton X-100, digitonin or the like), characteristics of the potassium channel may be maintained after the solubilization.

[4] The screening method of the present invention

It is possible to screen a substance suppressing the polypeptide of the present invention, using the cell of the present invention in which the polypeptide of the present invention is expressed.

As previously described, the polypeptide of the present invention is a potassium channel which is exclusively expressed in the hypothalamus. As described in Background Art, it is considered that a potassium channel is involved with the maintenance of a resting membrane potential, the repolarization of a membrane potential after depolarization, or the like. This shows that an agent for suppressing the potassium channel in neurons shifts the membrane potential to the depolarization side, and has an activity which increases the excitation of neurons.

Further, as described in Background Art, it is considered that an agent increasing the excitation of neurons in medial hypothalamic area causes a feeding-controlling activity. Therefore, it is considered that an agent for suppressing the potassium channel expressed in hypothalamus neurons is useful as an antiobestic agent by a feeding suppression in which the mechanism is an increase in the excitation of hypothalamus neurons.

Therefore, the cell of the present invention per se in which the polypeptide of the present invention is expressed may be used as a screening tool for an antiobestic agent (particularly an agent for suppressing the polypeptide of the present invention).

In this connection, to "suppress" the polypeptide of the present invention as used herein means to suppress the channel function, and includes suppressing the channel function by suppressing an expression of the polypeptide.

The method of the present invention for screening an antiobestic agent (particularly an agent for suppressing the polypeptide of the present invention) comprises the steps of:

bringing the cell of the present invention expressing the polypeptide of the present invention into contact with a compound to be tested; and
analyzing whether or not the polypeptide is suppressed.

Compounds to be tested which may be applied to the screening method of the present invention are not particularly limited, but there may be mentioned, for example, various known compounds (including peptides) registered in chemical files, compounds obtained by combinatorial chemistry techniques (Terrett, N. K. et al., Tetrahedron, 51, 8135-8137, 1995), or random peptides prepared by employing a phage display method (Felici, F. et al., J. Mol. Biol., 222, 301-310, 1991) or the like. In addition, culture supernatants of microorganisms, natural components derived from plants or marine organisms, or animal tissue extracts may be used as the test compounds for screening. Further, compounds (including peptides) obtained by chemically or biologically modifying compounds (including peptides) selected by the screening method of the present invention may be used.

The screening method of the present invention is not particularly limited, so long as it comprises the steps of:

bringing the cell of the present invention expressing the polypeptide of the present invention as an effective potassium channel (i.e., the cell which is transfected with an expression vector comprising the polynucleotide encoding the polypeptide of the present invention, and in which the polypeptide is expressed as an effective potassium channel) into contact with a compound to be tested; and
analyzing whether or not the polypeptide is suppressed. There may be mentioned, on the basis of differences in methods used for analyzing a suppression of the polypeptide of the present invention, for example,

[1] a screening method utilizing a voltage-clamp method (particularly whole-cell voltage-clamp method,

[2] a screening method utilizing a radioisotope rubidium ($^{86}$Rb$^+$) ion release, or

[3] a screening method utilizing a voltage-sensitive dye. Among these methods, the screening method utilizing a radioisotope rubidium ($^{86}$Rb$^+$) ion release is preferable.

When screening a substance which is useful as an antiobestic agent and suppresses the polypeptide of the present invention utilizing the voltage-clamp method (particularly whole-cell voltage-clamp method) of the item [1], it is analyzed whether or not the polypeptide of the present invention is suppressed, by voltage-clamping the cell of the present invention expressing the polypeptide of the present invention on the cell surface by the voltage-clamp method (particularly whole-cell voltage-clamp method) and analyzing (i.e., measuring or detecting) a whole-cell current of the cell in the presence of a test compound. That is, the screening method of the present invention utilizing the voltage-clamp method (particularly whole-cell voltage-clamp method) comprises the steps of:

bringing the cell of the present invention expressing the polypeptide of the present invention on the cell surface while voltage-clamping by the voltage-clamp method (particularly whole-cell voltage-clamp method), into contact with a test compound, and

analyzing a change of the whole-cell current in the cell.

More particularly, it is preferable to carry out the procedure by a method described in Example 4. For example, a solution containing 149 mol/L NaCl, 5 mmol/L KCl, 2 mmol/L MgCl$_2$, and 10 mmol/L HEPES-Na (pH = 7.3) may be used as an extracellular solution, and a solution containing 149 mmol/L KCl, 1.8 mmol/L MgCl$_2$, 4.5 mmol/L EGTA, and 9 mmol/L HEPES-K (pH = 7.3) may be used as an intracellular solution. For example, when a test compound is added to an extracellular solution of a voltage-clamped cell at a holding potential of -40 mV, and then an outward current is suppressed, it may be confirmed that the test compound is a substance which suppresses the polypeptide of the present invention.

When screening a substance which is useful as an antiobestic agent and suppresses the polypeptide of the present invention utilizing the radioisotope rubidium ($^{86}$Rb$^+$) ion release of the item [2], it is analyzed whether or not the polypeptide of the present invention is suppressed, by making the cell of the present invention expressing the polypeptide of the present invention on the cell surface incorporate the radioisotope rubidium ($^{86}$Rb$^+$) ion, and then analyzing (i.e., measuring or detecting) an amount of radioactivity released outside of the cell when a test compound is added (Maingret et al., J. Biol. Chem., 274, 1381, 1999). That is, the screening method of the present invention utilizing the radioisotope rubidium ($^{86}$Rb$^+$) ion release comprises the steps of:

making the cell of the present invention expressing the polypeptide of the present invention on the cell surface incorporate the radioisotope rubidium ($^{86}$Rb$^+$) ion, and then bringing the cell into contact with a test compound, and analyzing an amount of radioactivity released outside of the cell. This screening utilizes a feature that a potassium channel generally allows rubidium ions, as well as potassium ions, to pass through.

More particularly, it is preferable to carry out the procedure by a method described in Example 7. For example, the rubidium ($^{86}$Rb$^+$) ions can be incorporated into the cell of the present invention expressing the polypeptide of the present invention on the cell surface by incubating the cell with $^{86}$RbCl. The cell is washed with a solution containing a common concentration (such as 2.5 mmol/L) of potassium ions to remove rubidium ions not incorporated. The cell is stimulated with a high concentration (such as 100 mmol/L) of potassium ions, and then the rubidium ($^{86}$Rb$^+$) ions are released from the cell. When the potassium channel is suppressed, the amount of the rubidium ($^{86}$Rb$^+$) ion released from the cell decreases. Therefore, it can be analyzed whether or not the polypeptide of the present invention is suppressed, on the basis of the radioactivity in the extracellular solution as the channel activity. A substance which suppresses the polypeptide of the present invention can be screened by analyzing (i.e., measuring or detecting) the radioactivity released outside of the cell when a test compound is added.

When screening a substance which is useful as an antiobestic agent and suppresses the polypeptide of the present invention utilizing the voltage-sensitive dye of the item [3], it is analyzed whether or not the polypeptide of the present invention is suppressed, by making the cell of the present invention expressing the polypeptide of the present invention on the cell surface incorporate the voltage-sensitive dye, and then analyzing (i.e., measuring or detecting) a change of a fluorescence intensity thereof in the cell when a test compound is added. That is, the screening method of the present invention utilizing the voltage-sensitive dye comprises the steps of:

making the cell of the present invention expressing the polypeptide of the present invention on the cell surface incorporate the voltage-sensitive dye, and then bringing the cell into contact with a test compound, and analyzing a change of a fluorescence intensity thereof in the cell. This screening utilizes a feature that a change of a membrane potential by an opening of the potassium channel can be detected by the voltage-sensitive dye.

More particularly, DiBAC [bis-(1,3-dibutylbarbituric acid)trimethine oxonol; manufactured by Molecular Probe] or a derivative thereof may be used as the voltage-sensitive dye. The activity of the polypeptide of the present invention can be analyzed (i.e., measured or detected) using these dyes, and a substance which suppresses the polypeptide of the present invention can be screened by comparing changes of the fluorescence intensity of the dye in the presence and absence of a test compound. More particularly, when the potassium channel is suppressed, the fluorescence intensity increases.

Further, an antiobestic agent can be screened by selecting a substance for suppressing the polypeptide of the present invention using the method of the items [1] to [3].

[5] The antibody and the fragment thereof of the present invention

An antibody, such as a polyclonal antibody or a monoclonal antibody, which reacts with the polypeptide of the present invention may be obtained by directly administering the polypeptide of the present invention or a fragment thereof to various animals. Alternatively, it may be obtained by a DNA vaccine method (Raz, E. et al., Proc. Natl. Acad. Sci. USA, 91, 9519-9523, 1994; or Donnelly, J. J. et al., J. Infect. Dis., 173, 314-320, 1996), using a plasmid into which a polynucleotide encoding the polypeptide of the present invention is inserted.

The polyclonal antibody may be produced from a serum or eggs of an animal such as a rabbit, a rat, a goat, or a chicken, in which the animal is immunized and sensitized by the polypeptide of the present invention or a fragment thereof emulsified in an appropriate adjuvant (for example, Freund's complete adjuvant) by intraperitoneal, subcutaneous, or intravenous administration. The polyclonal antibody may be separated and purified from the resulting serum or eggs in accordance with conventional methods for polypeptide isolation and purification. Examples of the separation and purification methods include, for example, centrifugal separation, dialysis, salting-out with ammonium sulfate, or a chromatographic technique using such as DEAE-cellulose, hydroxyapatite, protein A agarose, and the like.

The monoclonal antibody may be easily produced by those skilled in the art, according to, for example, a cell fusion method of Kohler and Milstein (Kohler, G. and Milstein, C., Nature, 256, 495-497, 1975).

A mouse is immunized intraperitoneally, subcutaneously, or intravenously several times at an interval of a few weeks by a repeated inoculation of emulsions in which the polypeptide of the present invention or a fragment thereof is emulsified into a suitable adjuvant such as Freund's complete adjuvant. Spleen cells are removed after the final immunization, and then fused with myeloma cells to prepare hybridomas.

As a myeloma cell for obtaining a hybridoma, a myeloma cell having a marker such as a deficiency in hypoxanthineguanine phosphoribosyltransferase or thymidine kinase (for example, mouse myeloma cell line P3X63Ag8.U1) may be used. As a fusing agent, polyethylene glycol may be used. As a medium for preparation of hybridomas, for example, a commonly used medium such as an Eagle's minimum essential medium, a Dulbecco's modified minimum essential medium, or an RPMI-1640 medium may be used by adding properly 10 to 30% of a fetal bovine serum. The fused strains may be selected by a HAT selection method. A culture supernatant of the hybridomas is screened by a well-known method such as an ELISA method or an immunohistological method, to select hybridoma clones secreting the antibody of interest. The monoclonality of the selected hybridoma is guaranteed by repeating subcloning by a limiting dilution method. Antibodies in an amount which may be purified are produced by culturing the resulting hybridomas in a medium for 2 to 4 days, or in the peritoneal cavity of a pristane-pretreated BALB/c strain mouse for 10 to 20 days.

The resulting monoclonal antibodies in the culture supernatant or the ascites may be separated and purified by conventional polypeptide isolation and purification methods. Examples of the separation and purification methods include, for example, centrifugal separation, dialysis, salting-out with ammonium sulfate, or chromatographic technique using such as DEAE-cellulose, hydroxyapatite, protein A agarose, and the like.

Further, the monoclonal antibodies or the antibody fragments containing a part thereof may be produced by inserting the whole or a part of a gene encoding the monoclonal antibody into an expression vector and introducing the resulting expression vector into appropriate host cells (such as E. coli, yeast, or animal cells).

Antibody fragments comprising an active part of the antibody such as $F(ab')_2$, Fab, Fab', or Fv may be obtained by a conventional method, for example, by digesting the separated and purified antibodies (including polyclonal antibodies and monoclonal antibodies) with a protease such as pepsin or papain, and separating and purifying the resulting fragments by standard polypeptide isolation and purification methods.

Further, an antibody which reacts to the polypeptide of the present invention may be obtained in a form of single chain Fv or Fab in accordance with a method of Clackson et al. or a method of Zebedee et al. (Clackson, T. et al., Nature, 352, 624-628, 1991; or Zebedee, S. et al., Proc. Natl. Acad. Sci. USA, 89, 3175-3179, 1992). Furthermore, a humanized antibody may be obtained by immunizing a transgenic mouse in which mouse antibody genes are substituted with human antibody genes (Lonberg, N. et al., Nature, 368, 856-859, 1994).

EXAMPLES

The present invention now will be further illustrated by, but is by no means limited to, the following Examples. The procedures were performed in accordance with the known methods (for example, Maniatis, T., et al., "Molecular Cloning - A Laboratory Manual", Cold Spring Harbor Laboratory, NY, 1982; and Hille, B., Ionic Channels of Excitable Membranes, 2nd Ed., Sinauer Associates Inc., MA, 1992), unless otherwise specified.

Example 1: Isolation of gene encoding novel potassium channel and construction of expression vector

A full-length cDNA encoding the novel potassium channel of the present invention consisting of the amino acid sequence of SEQ ID NO: 2 was obtained by a reverse transcriptase-polymerase chain reaction (RT-PCR) using human brain poly A$^+$ RNA (Clontech) as a template by the following procedure.
First, a first-strand cDNA was synthesized by carrying out a reverse transcription from the human brain poly A$^+$ RNA (10 ng) as a template using a commercially available RT-PCR kit (SUPERSCRIPT First-Strand Synthesis System for RT-PCR; GIBCO-BRL).
A PCR was carried out using the resulting first-strand cDNA as a template, the oligonucletide consisting of the base sequence of SEQ ID NO: 3 (having the EcoRI recognition sequence added to the 5'-terminus) as a forward primed, the oligonucletide consisting of the base sequence of SEQ ID NO: 4 (having the BamHI recognition sequence added to the 5'-terminus) as a reverse primed, and DNA polymerase (PLATINUM Taq DNA Polymerase High-Fidelity; GIBCO-BRL). In the PCR, a thermal denaturation was first performed at 95°C for 5 minutes, and then a cycle composed of treatments at 95°C for 15 seconds and 68°C for 2 minutes and 30 seconds was repeated 35 times. As a result, a DNA fragment of approximately 1.6 kbp was amplified.
The resulting DNA fragment was digested with restriction enzymes EcoRI and BamHI, and cloned into a plasmid pIRESneo2 (CLONTECH). The resulting clone was named pIRESneo2-543. In this connection, the plasmid pIRESneo2 contains a cytomegalovirus promoter sequence and may be used for an expression of the novel potassium channel in an animal cell.
The base sequence of the resulting clone pIRESneo2-543 was analyzed using a DNA sequencer (ABI377 DNA Sequencer; Applied Biosystems) by a dideoxy terminator method, to obtain the base sequence of SEQ ID NO: 1.
The base sequence of SEQ ID NO: 1 contains an open reading frame consisting of 1632 base pairs (a sequence consisting of the 13th to 1644th bases in the base sequence of SEQ ID NO: 1). The amino acid sequence consisting of 543 amino acid residues deduced from the open reading frame was that of SEQ ID NO: 2.

Example 2: Analysis of expression distribution of novel potassium channel gene

An expression distribution of the gene encoding the novel potassium channel consisting of the amino acid sequence of SEQ ID NO: 2 in human tissues was analyzed by a RT-PCR method in accordance with the following procedure.
Poly A$^+$ RNA (5 ng, respectively; Clontech) from each human tissue was treated with DNase, and then a first-strand cDNA was synthesized by carrying out a reverse transcription using an RT-PCR kit (SUPERSCRIPT First-Strand Synthesis System for RT-PCR; GIBCO-BRL).
A PCR was carried out using the resulting first-strand cDNA as a template, the oligonucletide consisting of the base sequence of SEQ ID NO: 5 as a forward primed, the oligonucletide consisting of the base sequence of SEQ ID NO: 6 as a reverse primed, and DNA polymerase (PLATINUM Taq DNA Polymerase; GIBCO-BRL). In the PCR, a thermal denaturation was first performed at 95°C for 5 minutes, and then a cycle composed of treatments at 95°C for 30 seconds and 68°C for 2 minutes and 30 seconds was repeated 30 times. In this connection, the base sequences of the primers are those specific for the gene encoding the polypeptide consisting of the amino acid sequence of SEQ ID NO: 2.
When the RT-PCR analysis of each human tissue (amygdala, caudate nucleus, hippocampus, corpus callosum, substantia nigra, thalamus, cerebellum, frontal lobe, hypothalamus, spinal cord, pituitary, whole brain, heart, placenta, lung, trachea, liver, skeletal muscle, kidney, pancreas, small intestine, stomach, spleen, bone marrow, thymus, thyroid, salivary gland, adrenal gland, mammary gland, and prostate) was carried out, a DNA fragment of approximately 1.4 kbp was strongly amplified in the hypothalamus and weekly in the frontal lobe. It was found that the mRNA of the novel potassium channel of the present invention is exclusively expressed in the hypothalamus.

Example 3: Expression of novel potassium channel in animal cell L929 cell

The novel potassium channel of the present invention consisting of the amino acid sequence of SEQ ID NO: 2 was expressed in an animal cell to detect a channel activity of the protein. A L929 cell (ATCC: CRL-2148) in which a current from an endogenous channel is not generated by a change of its membrane potential was used as the animal cell. The L929 cell was transfected, using the expression vector pIRESneo2-543 obtained in Example 1 and a commercially available transfection reagent (LipofectAMINE; GIBCO-BRL), to express the potassium channel in the cell. In this connection, the concrete procedure was carried out in accordance with a manual attached to the transfection reagent. Further, a cell transfected with the plasmid pIRESneo2 was prepared as a control cell in a similar fashion.

The resulting transfected cells were used in the following Examples 4 and 5.

Example 4: Detection of channel activity of novel potassium channel

Each cell obtained in Example 3 was voltage-clamped and a whole-cell current was measured by a whole-cell voltage-clamp method. A solution containing 149 mmol/L NaCl, 5 mmol/L KCl, 2 mmol/L $MgCl_2$, and 10 mmol/L HEPES-Na (pH = 7.3) was used as an extracellular solution, and a solution containing 149 mmol/L KCl, 1.8 mmol/L $Mgd_s$-. 4.5 mmol/L EGTA, and 9 mmol/L HEPES-K (pH = 7.3) as an intracellular solution.

When a depolarization pulse was given to the cell transfected with the plasmid pIRESneo2-543 from a holding potential of -80 mV for 400 msec, an outward current was induced. This current contained instantaneous and delayed components, and inactivation was not observed during the voltage stimulus. When the same depolarization pulse was given to the control cell, such a current was not observed.

The results are shown in Fig. 1. In Fig. 1, Graph (A) shows the result obtained by using the L929 cell transfected with the plasmid pIRESneo2-543, and Graph (B) shows the result obtained by using the L929 cell transfected with the control vector (plasmid pIRESneo2).

Example 5: Confirmation of selectivity of potassium ion by novel potassium channel

To examine the selectivity of a potassium ion by the novel potassium channel of the present invention consisting of the amino acid sequence of SEQ ID NO: 2, each reversal potential was measured using a voltage ramp, while changing a potassium ion concentration in the extracellular solution from 5 mmol/L to various concentrations (15 mmol/L, 30 mmol/L, 75 mmol/L, and 150 mmol/L). As a result, when the potassium ion concentration was increased, the reversal potential was shifted to the depolarization side. The reversal potential in each potassium ion concentration was plotted and a linear regression was carried out. The slope was 49 mV/decade, which was close to the theoretical value of slope calculated from Nernst's equation. It is considered from this result that the novel potassium channel of the present invention consisting of the amino acid sequence of SEQ ID NO: 2 exhibits a high selectivity of the potassium ion.

Example 6: Expression of novel potassium channel in animal cell CHO-K1

The procedure described in Example 3 was repeated, except that a CHO-K1 cell was used as an animal cell, to express the potassium channel in the cell. These resulting transfected cells were used in the following Example 7.

Example 7: Detection of channel activity of novel potassium channel utilizing rubidium ($^{86}$Rb) ion release

To conveniently measure the potassium channel activity, an ion permeability of the novel potassium channel of the present invention consisting of the amino acid sequence of SEQ ID NO: 2 was examined by measuring a rubidium ion radioactivity in the novel potassium channel, on the basis of the feature that a potassium channel generally allows rubidium ions, as well as potassium ions, to pass through.

Each cell obtained in Example 6 was cultured in a culture liquid containing rubidium chloride ($^{86}$Rb chloride; 2 mCi/mL), and then rubidium ions were incorporated into the cell. Each cell was washed with a washing solution containing 150 mmol/L NaCl, 2.5 mmol/L KCl, 1.8 mmol/L $CaCl_2$, 0.8 mmol/L $MgCl_2$, and 5 mmol/L HEPES-Na (pH = 7.4) to remove rubidium ions not incorporated, and replaced with a measuring solution containing a high concentration of potassium ions [a solution containing 52.5 mmol/L NaCl, 100 mmol/L KCl, 1.8 mmol/L $CaCl_2$, 0.8 mmol/L $MgCl_2$, and 5 mmol/L HEPES-Na (pH = 7.4)]. When a rubidium ion radioactivity contained in the measuring solution was measured and analyzed by a liquid scintillation counter, the radioactivity was measured.

As a result, it is considered that the cell in which the novel potassium channel of the present invention is expressed allows rubidium ions to pass through due to a high potassium ion concentration stimulus (High K stimulus). It was confirmed in this system that the novel potassium channel of the present invention consisting of the amino acid sequence of SEQ ID NO: 2 exhibits the potassium channel activity.

Example 8: Analysis of expression distribution of novel potassium channel in human hypothalamus and frontal lobe

An expression distribution of the novel potassium channel consisting of the amino acid sequence of SEQ ID NO: 2 in human brain hypothalamus was analyzed.

As a polypeptide used for preparing an antibody, a polypeptide consisting of an amino acid sequence in which cysteine was added to the N-terminus of an amino acid sequence consisting of the 105th to 122nd amino acids in the amino acid sequence of SEQ ID NO: 2 was synthesized. The amino acid sequence consisting of the 105th to 122nd amino acids in the amino acid sequence of SEQ ID NO: 2 was determined by analyzing the amino acid sequence of SEQ ID NO: 2 using Hopp and Woods's algorithm. The prepared polypeptide was emulsified in an adjuvant, and then a rabbit was immunized and sensitized. A serum was obtained from the rabbit, and then a novel anti-potassium channel antibody was purified by a peptide affinity.

An immunohistochemical staining of human hypothalamus tissue section fixed by formalin and embedded with paraffin was carried out using the anti-potassium channel antibody diluted by a factor of 500 or 1000. In this connection, an antigen-antibody reaction was detected by an ABC method (Vector Laboratories's kit).

As a result of the immunohistochemical staining by the anti-potassium channel antibody, nucleus paraventricularis and supraoptic nucleus were strongly stained and nerve cell bodies in arcuate nucleus and ventromedial hypothalamic nucleas were stained, among hypothalamus, in both cases of dilution by a factor of 500 or 1000. Thalamus was negative.

It was found from this Example that the novel potassium channel consisting of the amino acid sequence of SEQ ID NO: 2 is expressed in nucleus paraventricularis, arcuate nucleus, and ventromedial hypothalamic nucleas, in which a leptin receptor is expressed.

Example 9: Analysis of expression distribution of novel potassium channel in mouse hypothalamus

An expression distribution of mouse potassium channel (ortholog) corresponding to the human potassium channel consisting of the amino acid sequence of SEQ ID NO: 2 in mouse brain hypothalamus was analyzed.

A male ddY mouse (purchased from Japan SLC, Inc.) was deeply anesthetized with sodium pentobarbital, and then its chest was opened. The right auricular appendage was incised, and then blood was drawn under reflux from the left ventricle with 20 mL of a phosphate buffer cooled with ice. Further, a fixation was carried out under reflux from the left ventricle with 20 mL of 100 mmol/L phosphate buffer (pH=7.4) containing 4% paraformaldehyde and 0.1% glutaric aldehyde cooled with ice. Its brain was quickly taken out and a fixation carried out at 4°C for 2 days using a 100 mmol/L phosphate buffer containing 4% paraformaldehyde. Further, it was immersed in a 100 mmol/L phosphate buffer (pH=7.4) containing 16% sucrose 4 times every three days to obtain mouse brain sucrose-fiexd sample. The brain tissue containing hypothalamus was placed on a sample stage, embedded with a compound (TISSUE-TEC; MILES), and quickly frozen by dry ice. Frozen sections having a thickness of 16 μm were prepared by a microtome (HM500-OM; Carl Zeiss).

A series of mouse hypothalamus sections prepared at intervals of 160 μm were treated with a 100 mmol/L phosphate buffer (pH=7.4) containing 2% hydrogen peroxide and 0.3% Triton X-100 at room temperature for 30 minutes, were blocked with 10% normal goat serum (Vector Laboratories's kit), and were reacted with the anti-potassium channel antibody prepared in Example 8 and diluted by a factor of 30000 with a 100 mmol/L phosphate buffer (pH=7.4) containing 1% normal goat serum and 0.3% Triton X-100 at 4°C for 3 days. An antigen-antibody reaction was detected by an ABC method (Vector Laboratories's kit). Further, Nissl staining of serial sections was carried out, and each nucleus in mouse hypothalamus was isolated in accordance with Keith B. J. F. et al., "The Mouse Brain in Stereotaxic Coordinates", Academic Press, NY, 1996. When an absorption experiment was carried out using the polypeptide prepared in Example 8, the staining was inhibited by adding 0.1 μmol/L polypeptide in the antibody reaction.

As a result of the expression analysis using the novel anti-potassium channel antibody prepared in Example 8, nerve cell bodies around the hypothalamus were stained in mouse hypothalamus. Many of the stained neurons were of a small size. A precursor cell of oligodendrocyte having a thorn-like projection around its cell body was stained.

The expression of the novel potassium channel in hypothalamus was strongly observed in ventromedial hypothalamic nucleas. In particular, it was strongly expressed in the outer area of ventromedial hypothalamic nucleas. Further, a relatively strong expression was observed in arcuate nucleus, tuberal nucleus, supraoptic nucleus, and nucleus paraventricularis. By contrast, anterior hypothalamic area, lateral hypothalamic area, lateroanterior hypothalamic nucleas, dorsomedial hypothalamic nucleus, and suprachiasmatic nucleus were negative except for a part thereof.

It was found from this Example that the mouse potassium channel corresponding to the human potassium channel consisting of the amino acid sequence of SEQ ID NO: 2 is expressed in nucleus paraventricularis, arcuate nucleus, and ventromedial hyplthalamic nucleas, in which a leptin receptor is expressed.

Example 10: Expression of novel potassium channel in animal cell CHOdhfr

To express the novel potassium channel of the present invention consisting of the amino acid sequence of SEQ ID NO: 2, the polypeptide was expressed in an animal cell. A dihydrofolate reductase defective strain (CHOdhfr- cell) of a Chinese hamster ovary cell (CHO) was used as the cell. The CHOdhfr- cell was transfected, using the expression vector pIRESneo2-543 obtained in Example 1 and a commercially available transfection reagent (LipofectAMINE2000; Invitrogen), to express the potassium channel in the cell. As a plate for cell culture, a 96 well plate [Cytostar-T Scintillating Microplates (RPNQ0163), Amersham Pharmacia Biotech; 96F Nunclon Delta White microwell (136102), Nalge Nunc; or Culture Plate (white) (6005180), Packard Japan], or a 24 well plate for cell culture [Microplate (3820-024N), ASAHI TECHNO GLASS CORPORATION; or Culture Plate (white) (6005180), Packard Japan] was used. In this connection, the concrete procedure was carried out in accordance with a manual attached to the transfection reagent. These resulting transfected cells were used in the following Example 11.

Example 11: Screening substance inhibiting novel potassium channel utilizing radioisotope rubidium ($^{86}$Rb$^+$) ion release

A substance inhibiting an activity of the novel potassium channel of the present invention consisting of the amino acid sequence of SEQ ID NO: 2 was screened by measuring a rubidium ion radioactivity, on the basis of the feature that a potassium channel generally allows rubidium ions, as well as potassium ions, to pass through.

Each cell obtained in Example 10 was cultured in a culture liquid containing rubidium chloride ($^{86}$Rb chloride; 2 mCi/mL), and then rubidium ions were incorporated into the cell. Each cell was washed with a washing solution containing 150 mmol/L NaCl, 2.5 mmol/L KCl, 1.8 mmol/L CaCl$_2$, 0.8 mmol/L MgCl$_2$, and 5 mmol/L HEPES-Na (pH = 7.4), or with a commercially available medium [Minimum Essential Medium Alpha Medium (Cat. 12571-041); Invitrogen] to remove rubidium ions not incorporated. Then, it was replaced with a measuring solution A containing a high concentration of potassium ions [52.5 mmol/L NaCl, 100 mmol/L KCl, 1.8 mmol/L CaCl$_2$, 0.8 mmol/L MgCl$_2$, and 5 mmol/L HEPES-Na (pH = 7.4)], a measuring solution B containing arachidonic acid and a high concentration of potassium ions [52.5 mmol/L NaCl, 100 mmol/L KCl, 1.8 mmol/L CaCl$_2$, 0.8 mmol/L MgCl$_2$, 5 mmol/L HEPES-Na (pH=7.4), and 100 µmol/L arachidonic acid], or solutions in which a test compound solution [i.e., a solution in which a test compound had been dissolved in dimethyl sulfoxide (DMSO)] had been added to these measuring solutions (i.e., the measuring solution A or the measuring solution B), and then each stimulation was carried out for 15 minutes. Further, as a negative control, it was again replaced with the washing solution, and stimulation was carried out for 15 minutes. After the stimulation, each solution was transferred to a 96 well [OptiPlate (6005190); Packard Japan] or 24 well [OptiPlate (6005186); Packard Japan] plate for measurement. The remaining cell on each plate was dissolved in 0.2 mol/L NaOH, and appropriately. transferred to a measuring plate. The rubidium ion radioactivity contained in each well of the plate was measured by a multi-well scintillation counter (TopCount NXT HTS; Packard).

INDUSTRIAL APPLICABILITY

The polypeptide of the present invention is a potassium channel which is exclusively expressed in the hypothalamus, and which is expressed, among the hypothalamus, in the tissue in which a leptin receptor having activities suppressing feeding and inducing an energy consumption is similarly expressed. Therefore, an antiobestic agent can be obtained by screening a substance for suppressing the polypeptide of the present invention.

Further, according to the cell of the present invention expressing the polypeptide of the present invention on the cell surface, a convenient screening system for the antiobestic agent can be provided. Furthermore, the polynucleotide, expression vector, cell, and antibody of the present invention are useful in producing the polypeptide of the present invention.

FREE TEXT IN SEQUENCE LISTING

Features of "Artificial Sequence" are described in the numeric identifier <223> in the Sequence Listing. More particularly, each of the base sequences of SEQ ID NOS: 3 and 4 is an artificially synthesized primer sequence.

As above, the present invention is explained with reference to particular embodiments, but modifications and improvements obvious to those skilled in the art are included in the scope of the present invention.

**Claims**

1. (1) A polypeptide consisting of an amino acid sequence of SEQ ID NO: 2, or (2) a polypeptide exhibiting a potassium channel activity and consisting of an amino acid sequence in which one or several amino acids are substituted, deleted, inserted, and/or added in an amino acid sequence of SEQ ID NO: 2.

2. A polypeptide consisting of an amino acid sequence of SEQ ID NO: 2.

3. A polynucleotide encoding the polypeptide according to claim 1 or 2.

4. An expression vector comprising the polynucleotide according to claim 3.

5. A cell transfected with the expression vector according to claim 4.

6. A method for screening an agent for suppressing the polypeptide according to claim 1 or 2, comprising the steps of:

   bringing a cell expressing said polypeptide into contact with a compound to be tested; and
   analyzing whether or not said polypeptide is suppressed.

7. A method for screening an antiobestic agent, comprising the steps of:

   bringing a cell expressing the polypeptide according to claim 1 or 2 into contact with a compound to be tested; and analyzing whether or not said polypeptide is suppressed.

8. A process for producing the polypeptide according to claim 1 or 2, comprising the steps of:

   culturing the cell according to claim 5; and
   recovering said polypeptide.

9. An antibody or a fragment thereof, which binds to the polypeptide according to claim 1 or 2.

F I G. 1

(A)

1nA

100ms

(B)

# INTERNATIONAL SEARCH REPORT

| International application No. |
|---|
| PCT/JP01/11330 |

**A. CLASSIFICATION OF SUBJECT MATTER**
Int.Cl$^7$   C12N15/09, C12N15/12, C07K14/705, C12N1/15, C12N1/19, C12N1/21, C12N5/10, C12Q1/02, C07K16/18

According to International Patent Classification (IPC) or to both national classification and IPC

**B. FIELDS SEARCHED**

Minimum documentation searched (classification system followed by classification symbols)
Int.Cl$^7$   C12N15/09, C12N15/12, C07K14/705, C12N1/15, C12N1/19, C12N1/21, C12N5/10, C12Q1/02, C07K16/18

Documentation searched other than minimum documentation to the extent that such documents are included in the fields searched

Electronic data base consulted during the international search (name of data base and, where practicable, search terms used)
SwissProt/PIR/GeneSeq, MEDLINE (STN), Genbank/EMBL/DDBJ/GeneSeq, WPI (DIALOG), BIOSIS (DIALOG)

**C. DOCUMENTS CONSIDERED TO BE RELEVANT**

| Category* | Citation of document, with indication, where appropriate, of the relevant passages | Relevant to claim No. |
|---|---|---|
| X<br>Y | Florian Lesage et al., "Human TREK2, a 2P Domin Mechano-sensitive K$^+$ Channel with Multiple Regulation by Polyunsaturated Fatty Acids, Lysophospholipids, and G$_s$, G$_i$, and G$_q$ Protein-coupled Receptors", The Journal of Biological Chemistry, September, 2000, Vol.275, No.37, pages 28398 to 28405 | 1-6,8-9<br>7 |
| Y | D. SPANSWICK et al., "Insulin activates ATP sensitive K$^+$ channels in hypothalamic neurons of lean, but not obese rats", Nature Neuroscience, August, 2000, Vol.3, No.8, pages 757 to 758 | 7 |
| P,A | WO 01/85788 A2 (Pharmacia & Upjohn Company), 15 November, 2001 (15.11.2001), (Family: none) | 1-9 |
| A | Hyoweon BANG et al., "TREK-2 a New Member of the Mechanosensitive Tandem-pore K$^+$ Channel Family", The Journal of Biological Chemistry, June, 2000, Vol.275, No.23, pages 17412 to 17419 | 1-9 |

☐ Further documents are listed in the continuation of Box C.      ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 28 January, 2002 (28.01.02) | 12 February, 2002 (12.02.02) |

| Name and mailing address of the ISA/<br>    Japanese Patent Office | Authorized officer |
|---|---|
| Facsimile No. | Telephone No. |

Form PCT/ISA/210 (second sheet) (July 1992)